(19) Europäisches Patentamt
European Patent Office
Office européen des brevets

(11) **EP 4 116 990 A1**

(12) **EUROPEAN PATENT APPLICATION**

(43) Date of publication:
**11.01.2023 Bulletin 2023/02**

(21) Application number: **22184190.1**

(22) Date of filing: **11.07.2022**

(51) International Patent Classification (IPC):
*G16H 20/30* (2018.01)   *A63B 24/00* (2006.01)
*A61B 5/00* (2006.01)   *A61B 5/11* (2006.01)
*G06K 9/00* (2022.01)   *G06V 40/20* (2022.01)

(52) Cooperative Patent Classification (CPC):
**G16H 20/30; A61B 5/1114; A61B 5/486;
A61B 5/6806; G06K 9/00496; G06V 40/23;**
A63B 2220/17; A63B 2220/34; A63B 2220/40;
A63B 2220/56

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.07.2021 PL 43839821
06.03.2022 EP 22160363**

(71) Applicant: **SHAPE.CARE Sp. z o.o.
30-394 Krakow (PL)**

(72) Inventors:
• **KRZYSZKOWSKI, Tomasz
31-207 Krakow (PL)**

• **HOLUBOWICZ, Piotr
31-348 Krakow (PL)**
• **ZUZIAK, Axel
31-352 Krakow (PL)**
• **SIWEK, Michal
31-834 Krakow (PL)**
• **KUCHARZ, Piotr
30-410 Krakow (PL)**
• **CHWALEK, Kamil
31-236 Krakow (PL)**

(74) Representative: **Kancelaria Eupatent.pl Sp. z.o.o
Ul. Kilinskiego 185
90-348 Lodz (PL)**

(54) **A METHOD AND A SYSTEM FOR MONITORING OF EXERCISES PERFORMED IN GLOVES**

(57)   A method for monitoring of exercises performed by a user, the method comprising providing the user with a pair of gloves (110, 120), wherein each of the gloves (110, 120) comprises a measurement module (130). The method comprises performing the following steps, in real time, while the user performs the exercise while wearing the gloves (110, 120): at each glove, during performance of the exercise set, reading and pre-processing (201) measurement data from the sensors (132) and accelerometer (133) in the gloves (110, 120) to determine at least a standardized total force measured by the sensors (132) during performance of the exercise set; and by means of the signaling system (143), providing a feedback (204) to the user, wherein the feedback indicates at least a balance between the standardized total force measured at the left glove (110) and the right glove (120).

Fig. 1

EP 4 116 990 A1

**Description**

TECHNICAL FIELD

The present invention relates to monitoring and analyzing of exercises performed in gloves, in particular strength workout exercises, such as weightlifting.

BACKGROUND

Weightlifting is a popular form of both professional and recreational workout. Exercisers can track their training progress by recording various parameters, such as the number of repetitions or lifted weight, in order to improve their results. In addition, in order to prevent injuries and to optimize the added benefits of training, the exercisers can monitor exercise performance to ensure proper technique or to select appropriate exercise regimens. However, since it can be difficult for the exercisers to self-assess their own performance in weightlifting without external reference or evaluation, immediate feedback during training would improve the effectiveness of training regimens. Observation of the training is usually carried out by a personal trainer who may instruct the exerciser with suggestions on how to properly perform the exercise. However, such solution is available only for a small group of exercisers, due to the time and costs associated with the involvement of a personal trainer.

There are various types of automated training monitoring systems that use various types of electronic devices.

A US patent application US2017216665A1 describes a system that uses sensors mounted on exercise equipment. However, such system requires use of exercise equipment that must be adapted in a predetermined manner.

A US patent application US20150196803A1 describes a video monitoring system that processes an image of the exerciser and analyzes the exercise on a regular basis, providing appropriate instructions. However, such system requires lots of computing power to process the data.

A US patent application US2018193699A1 describes special gloves with sensors to monitor the number of movements performed by a manual labor worker, but the measurement precision and method of use is not suitable for accurate monitoring of performance of physical exercises.

SUMMARY OF THE INVENTION

There is a need to provide a method of monitoring of exercises, to constantly inform the user about the quality of performance of the exercises in real time, while the preforming the exercises.

In one aspect, the invention relates to a method for monitoring of exercises performed by a user. The method comprises providing the user with a pair of gloves including a left glove and a right glove, wherein each of the gloves comprises a measurement module, the measurement module comprising: a three-axis accelerometer; a three-axis gyroscope; at least four pressing force sensors arranged at predetermined positions, wherein the positions of the pressing force sensors on the left glove are symmetrical with respect to the positions of the pressing force sensors on the right glove; and a controller for collecting measurement data from the sensors and the accelerometer; a glove wireless data transmission module. The method further comprises providing a data processing device, the data processing device comprising: a wireless data transmission module communicatively coupled to the glove wireless data transmission modules of each of the gloves; a data processing system; and a signaling system. The method comprises performing the following steps, in real time, while the user performs the exercise while wearing the gloves : at each glove, during performance of the exercise set, reading and pre-processing measurement data from the sensors and accelerometer in the gloves to determine at least a standardized total force measured by the sensors during performance of the exercise set; by means of the glove wireless data transmission modules of each of the gloves, transmitting the pre-processed data to the wireless data transmission module of the data processing device; by means of the data processing system, processing the transmitted data to analyze differences between data from the sensors of the left glove and the data from the sensors of the right glove; and by means of the signaling system, providing a feedback to the user, wherein the feedback indicates at least a balance between the standardized total force measured at the left glove and the right glove.

The data processing device can be a smartphone operating an application executing the functions of the data processing circuit.

The data processing device can be a tablet or a TV set operating an application performing the functions of the data processing circuit.

The signaling system may comprise a display, an audio emitter or a vibrating element embedded within the glove.

The glove may comprise a pulse oximeter configured to measure the pulse and blood oxygen saturation of the user.

Each glove may comprise four pressing force sensors in the following arrangement: a first sensor arranged at the middle finger near the middle phalanx bone, a second sensor arranged at the index finger near the proximal phalanx bone, a third sensor arranged at the second metacarpal bone and a fourth sensor arranged at the fifth metacarpal bone, when the glove is positioned on the hand of the user.

The method may further comprise: before reading and pre-processing measurement data from the sensors and the accelerometer, detecting a finger tap on the glove; and in response to detecting the finger tap, starting reading of the transmitted data from the pressing sensors and the accelerometer and continuing the reading until detecting another finger tap.

At least one glove can be provided with a pedometer.

The measurement module may comprise a computing module for preprocessing the sensor data.

The reading and pre-processing measurement data from the sensors may comprise storing measurement data read from the sensors and pre-processing stored measurement data after the exercise is stopped to determine a total pressing force measured by the sensors and sending a final set of pre-processed data.

The reading and pre-processing measurement data from the sensors may comprise storing measurement data read from the sensors and repetitively pre-processing stored measurement data to determine a total pressing force measured by the sensors and sending a partial set of pre-processed data.

In another aspect, the invention relates to a system for monitoring of exercises performed in gloves, comprising a pair of gloves and a data processing device having a structure and configured to operate as described herein.

The presented method allows to check whether a user performs the exercises correctly. In particular, by measuring the distribution of forces acting on gloves (i.e., force measured by individual pressing force sensors), it can be checked whether the user correctly grips (i.e., places the hand on the grip) and lifts weights. Moreover, by measuring the positioning plane (using an accelerometer) of the hand, it allows to determine anomalies and errors in work of hands while exercising. By comparing the data from the sensors of the left and right glove, it allows to determine the balance of forces between the left hand and the right hand. As a result, when presenting the data to the user, the user can be informed, for example, to correct the position of one hand in relation to the other hand for better exercise results, in particular so as to distribute the weight more evenly being lifted between the right and left hand.

By analyzing the values of signals from the sensors over time, the method can be used to recognize individual repetition cycles of exercise, i.e., the beginning and end of the repetition cycle. As a result, it is possible to determine the duration of the repetition, the number of repetitions in a set and the time interval between the individual repetitions. The results of the analysis of the change in duration of each repetition over several sets are presented to the user. This information makes it possible to evaluate the dynamics of the performed sets and by comparing it with the previous set or typical values for a given exercise stored in the device's memory, it is possible to assess the quality of the performed exercises in relation to previously performed exercises.

In particular, the length of the intervals between the repetitions and between the sets allows to determine the degree of the tiredness of the user.

The three-axis accelerometer detects the change in the position of the hand during the exercise, and the pressing force sensors detect the pressing force corresponding to the lifted load on individual locations on the hand.

Thanks to the use of a three-axis gyroscope, it is possible to detect the angular position of the user's hand while exercising.

The wireless interface can be an interface compatible with one of the common data transmission standards, for example Bluetooth, Wi-Fi, or an own transmission system dedicated to a given system.

The data processing device may be a smartphone that operates an application handling the functions of the data processing system. The smartphone provides a communication interface (for example, Bluetooth) and computing power to process sensor data, and allows the measurement data and the results of the analysis of the measurement data to be stored to create an exercise and training history. In this way, the functionality of the system can be made available to a wide range of smartphone users.

The data processing device may be also a tablet or a TV set executing an application that performs the functions of a data processing system. The tablet or the TV set may constitute an equipment assigned to the exercise performance location (for example, in a gym, each exercise station may have a dedicated tablet or TV installed) to enable the use of the system by users who do not have their own smartphone with the application or to provide feedback to users via a relatively large screen.

It is also possible to provide dedicated terminals to support the functionality of the data processing device.

The signaling system may comprise a display. By means of the display, the user can be informed about the current course of the exercises, suggestions for improving the performance of these exercises, etc. The information can be displayed by means of text or graphics, for example in the form of graphs.

The signaling system may comprise a sound emitter. By means of the sound emitter, the user can get a feedback in the form of voice commands or tone signals. Then the user does not need to observe the display. The sound emitter can be a loudspeaker that emits sound to the environment, or it can be in the form of earphones or headphones.

The signaling system may include a vibrating element built into the glove. By means of the vibrating system, the user can get a feedback, suggesting, for example, to increase the intensity of the work of the weaker hand (for example, by vibrating the element in the particular glove at a certain rhythm).

Alternatively, it is possible to use a vibrating element which is already present on the smartphone to provide feedback to the user. For example, the smartphone may be in the exerciser's pocket, and the information on the right hand is signaled with a different rhythm than the information on the left hand.

The glove may comprise a pulse oximeter to measure the pulse rate and blood oxygen saturation.

Each glove may contain four pressing force sensors located respectively on the middle finger near the middle phalanx bone, on the forefinger near the proximal phalanx, near the second metacarpal bone and near the fifth metacarpal bone. The present inventors have found that such placing of the sensors allows for optimal monitoring of most of the popular weight exercises discussed in the embodiments.

The accelerometer may be adapted to detect a finger tap (such as a single tap or a double tap, or a directional tap (having predetermined parameters in X, Y or Z axis)) on a glove. This allows the user to intuitively control the system by issuing appropriate commands corresponding to the type of tap, for example a weaker or stronger tap, single or multiple, short, or long-held taps. These commands can activate various system functions, such as starting and stopping a measurement (to optimize battery life), displaying historical exercise results, etc.

At least one glove may comprise with a pedometer so that it is possible to determine the number of steps taken by the user. In addition, by providing parameters that allow to determine the length of the user's step, it is possible to calculate the length of the route that the user has made.

The measurement module may include a computing module for preprocessing the sensor data. Owing to this, the algorithms perform the initial data processing and draw conclusions from the measurements, while wearing the glove, in the measurement module, and the application (for example, an application on a smartphone) draws wider and more accurate conclusions on their basis by analyzing data from many devices and data from previous sets.

The invention also corresponds to a system for monitoring of exercises performed in gloves, comprising a pair of gloves and a data processing device (140) having a structure and configured to operate as described above.

These and other features, aspects and advantages of the invention will become better understood with reference to the following drawings, descriptions, and claims.

BRIEF DESCRIPTION OF DRAWINGS

The present invention is shown by means of preferable embodiments in a drawing, wherein:

Fig. 1 shows a block diagram of an exercise performance monitoring system;
Fig. 2 shows the steps of a method for exercise performance monitoring;
Fig. 3A shows a glove with a sensor system;
Fig. 3B shows the arrangement of the measurement points on the inner side of the hand;

Fig. 4 shows the sensor system in cross-section;
Fig. 5 is an exploded view of the sensor system;
Fig. 6A shows the flexible mount for the sensor system;
Fig. 6B shows a flexible strip with conductive tracks;
Fig. 6C shows a 'pogo' type connector;
Fig. 7A shows schematic top view of a fabric glove with conductive threads;
Fig. 7B shows schematically of a woven glove with an exemplary arrangement of pressing force sensors on the inner side of the glove;
Fig. 8 shows an example reading of data from accelerometers during an exercise of lifting a dumbbell sideways while standing;
Fig. 9 shows an example of reading data from a single pressing force sensor on the left and right gloves during an exercise involving lifting dumbbells sideways while standing;
Fig. 10 shows an example reading of data from accelerometers during a deadlift exercise;
Fig. 11 shows an exemplary reading of data from a single pressing force sensor on the left and right gloves during a deadlift exercise;
Fig. 12 shows an example reading of accelerometer data during a dumbbell lifting exercise for a biceps exercise;
Fig. 13 shows an exemplary reading of data from a single pressing force sensor on the left and right gloves during a dumbbell lifting exercise for a biceps exercise;
Fig. 14 shows the distribution of forces acting on the hand when performing certain types of exercises to indicate which sensors play the most important role in monitoring the particular exercise;
Fig. 15A shows a first embodiment of a procedure for reading and pre-processing measurement data;
Fig. 15B shows a second embodiment of a procedure for reading and pre-processing measurement data;
Fig. 16 presents an example of a summary message.

DETAILED DESCRIPTION

The description presented herein is not to be taken in a limiting sense but is made merely for the purpose of illustrating the general principles of the invention.

The embodiment presented herein contains all features foreseen by the present disclosure. However, other embodiments are feasible as well that do not contain all features necessary to achieve particular technical advantages corelated with such feature.

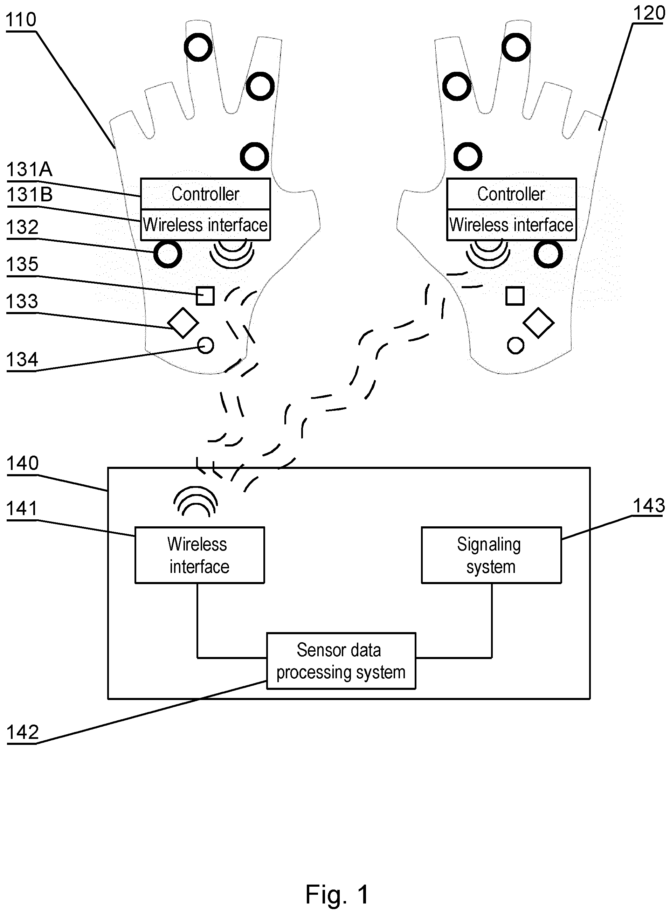

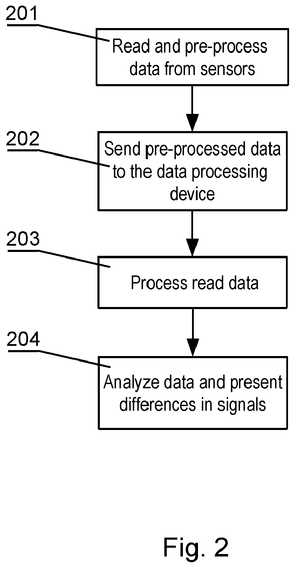

Fig. 1 shows a block diagram of an exemplary exercise performance monitoring system with which the exercise performance monitoring method shown in Fig. 2 may be implemented.

The user performs the exercises with a pair of gloves: a left glove 110 and a right glove 120. Each glove comprises a measurement module 130 which includes a three-axis accelerometer 133, a three-axis gyroscope 134, at least four pressing force sensors 132 (arranged at certain positions symmetrically on the left glove 110 with respect to the right glove 120), a controller 131A for collecting measurement data from the sensors 132, 133, 134 and a wireless interface 131B. The gloves communicate with a data processing device 140 which includes a wireless interface 141 communicatively connected with the wireless data interfaces 131B of the gloves 110, 120, a data processing circuit 142 and signaling system 143.

During performance of the exercises by the user wearing gloves 110, 120, the method comprises the steps of reading and pre-processing (step 201) measurement data from the sensors 132, 133, 134 in real time in the controller 131A, transmitting (step 202) the pre-processed data to the data processing device 140, and processing (step 203) the transmitted data, to i.a. analyze differences in the data from the sensors in the left glove 110 with respect to the data from the sensors in the right glove 120. By means of the signaling circuit 143 the feedback is communicated (step 204) to the user depending on the detected differences.

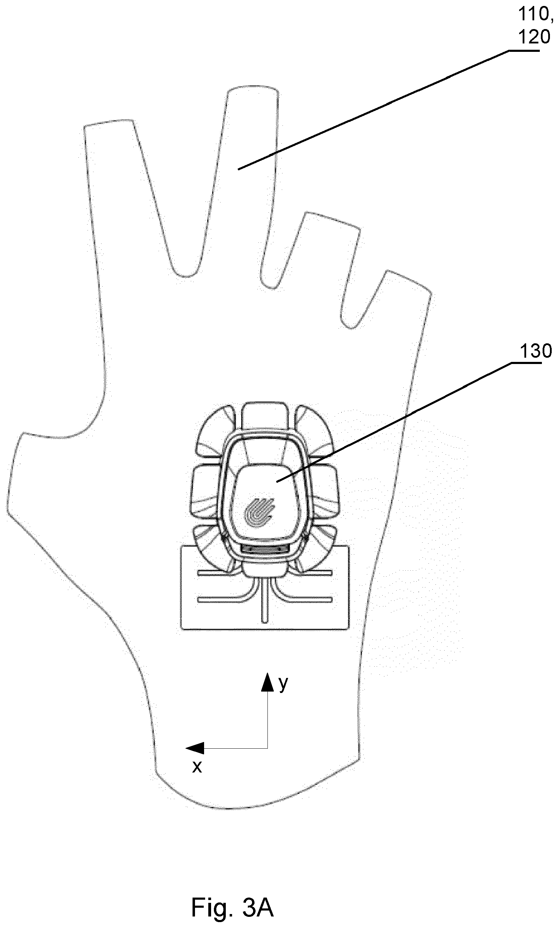

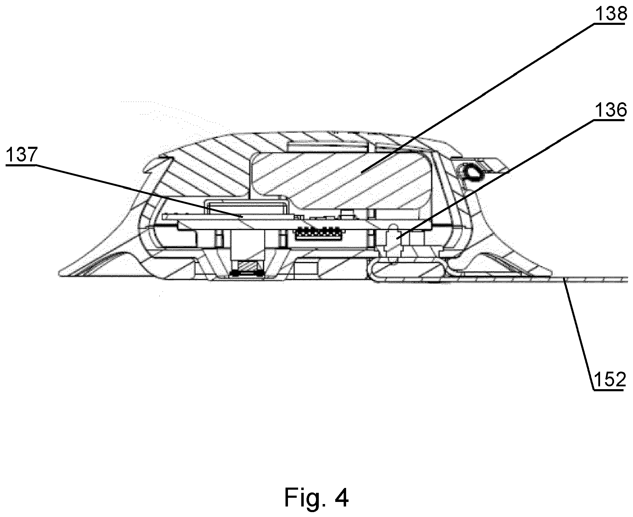

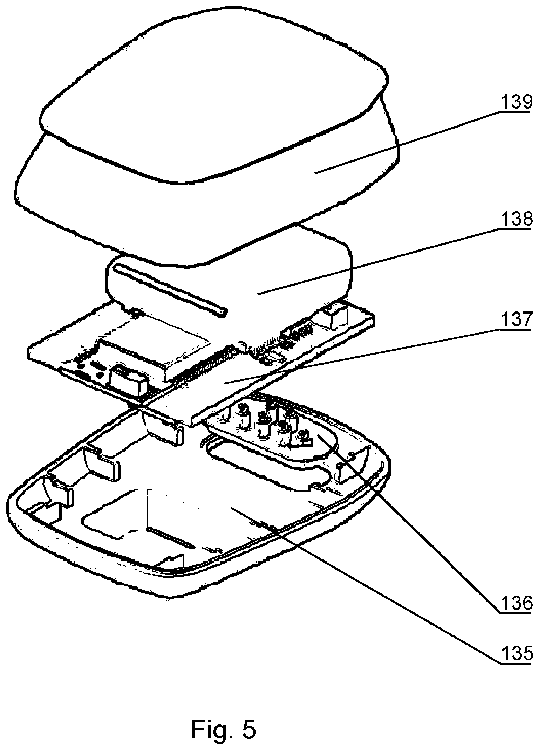

The measurement module 130 constituting the sensor system may have a form of a PCB (printed circuit board) 137 to which by means of flexible conductive threads and by means of the connectors 136 pressing force sensors 132 are connected. Preferably, an accelerometer 133 is provided on a printed circuit board 137. The measurement module 130 is powered by a battery 138, which, along with the circuit board 137, is located in a housing that includes a lower portion 135 and an upper portion 139 as shown in Figs. 3A, 4, and 5.

The design of the housing protects the measurement module 130 against accidental crushing (e.g., when a glove with the module lying on the ground is stepped on) or hitting a hard surface. Since the PCB 137 with the accelerometer 133 is rigidly mounted within the housing 135, 139, it is possible to effectively detect haptic commands from a user, such as tapping the housing to activate specific functions of the measurement module 130.

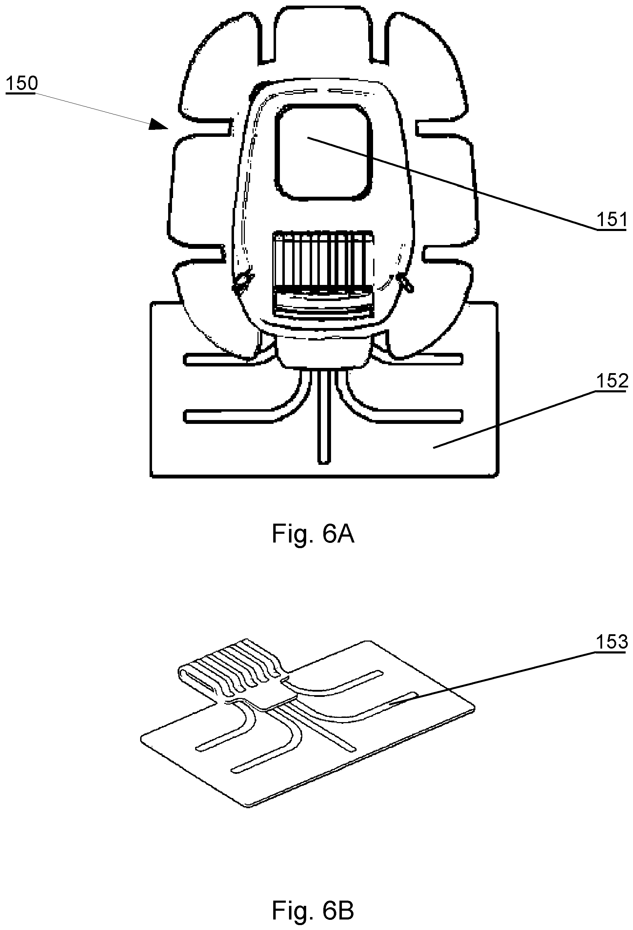

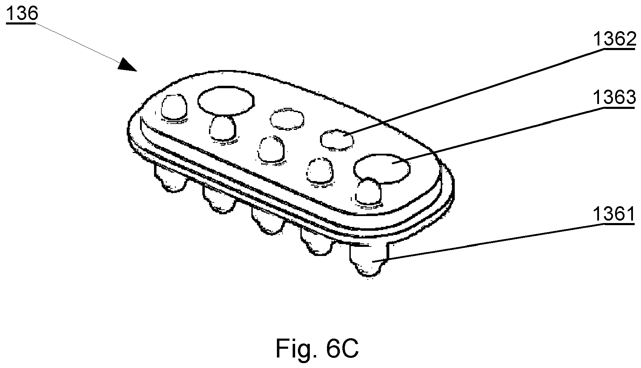

As shown in Figs. 6A-6C, the connector 136 preferably is in the form of a 'pogo' type connector in which resilient communication pins 1361 abut a flexible band 152 with conductive tracks 153 to which conductive threads from sensors are connected. In addition, the connector 136 has two charging pins 1362 of the measurement module 130, and magnets 1363 for attaching the charger plug. The glove has a pulse oximeter attached to the circuit board 137 such that its measurement beam passes towards the user's skin through the pulse oximeter opening 151 located in the flexible mount 150.

Preferably, the measurement module 130 along with the housing 135, 139 is located in a flexible mount 150, which in turn is attached to the glove 110, 120 e.g., by glue or is sewn into the material of the glove (or placed in a special pocket in the glove material). As a result, the measuring module 130 can be easily separated from the glove 110, 120, e.g., for the purpose of washing gloves.

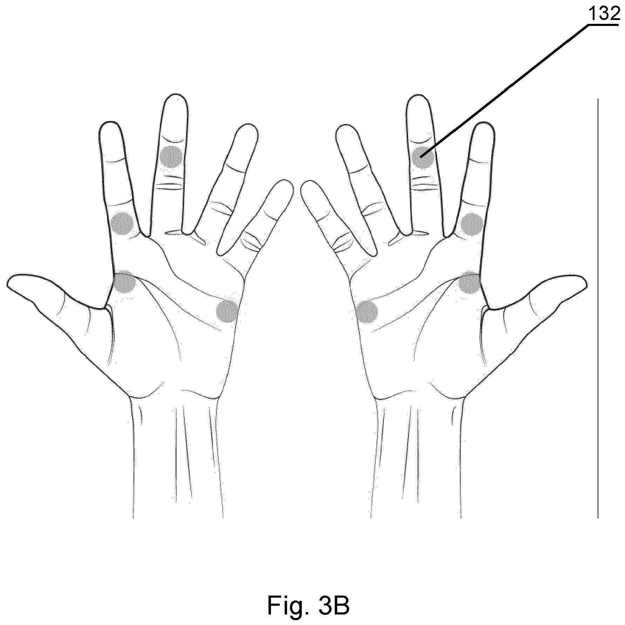

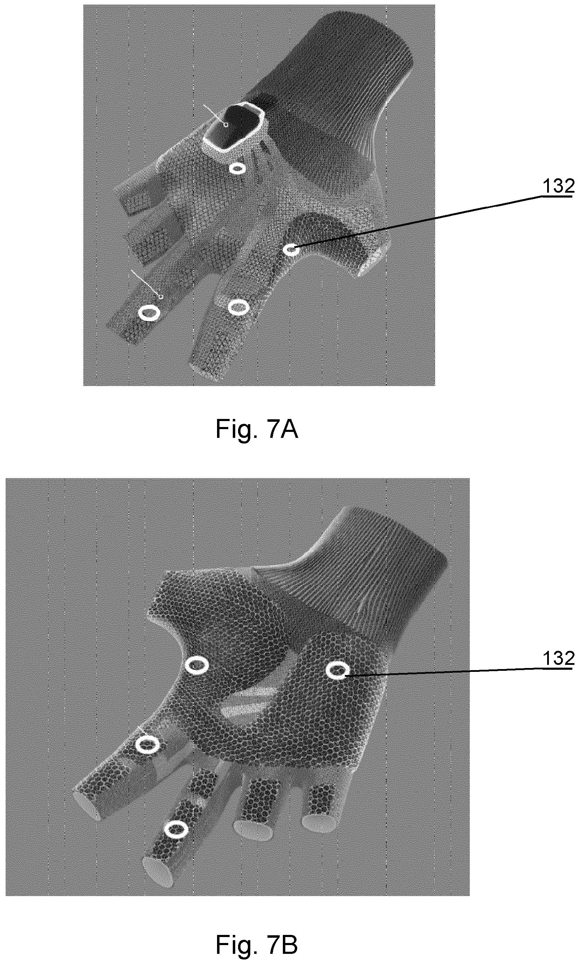

An exemplary arrangement of pressing force sensors 132 on the inside of the glove is shown in Figs. 7A and 7B. Fig. 3B shows the arrangement of the measurement points on the inner side of the hand.

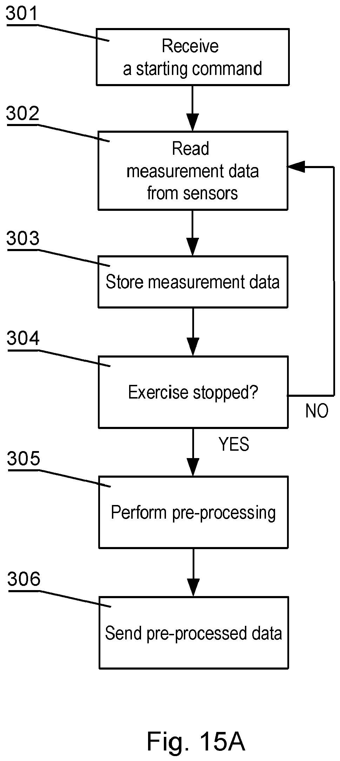

Fig. 15A shows a first embodiment of a procedure for reading and pre-processing measurement data read from the sensors 132, 133 by the controller 131A.

The procedure is initiated in step 301 by receiving a starting command to start measurement of exercise set. The starting command may be initiated by the user using an interface of an application executed at the data processing device, e.g., by pressing a dedicated EXERCISE START button. Alternatively, the starting command may be initiated by a haptic command input to the measurement module 130, e.g., a tap or double tap command. The data processing device may play an audible sound informing the user about receipt of the starting command.

Steps 302-303 are performed in a loop with a set frequency, e.g., 10 Hz or 100 Hz, depending on measurement precision accuracy. The steps may be performed with a different frequency for different sensors, e.g., the measurements of the acceleration sensor 133 may be read with a higher frequency than the measurements of the pressing force sensors 132.

In step 302 measurement data from the sensors is read and in step 303 the read measurement data is stored in memory of the controller. The measurement data that is stored may represent a direct value of signal (e.g., voltage value) read from the sensor or a value converted to some units of measure (e.g., a voltage value converted to acceleration or pressing force). Therefore, for each iteration of the loop 302-303, a set of values representing e.g., acceleration in X axis, acceleration in Y axis, acceleration in Z axis, pressing force read by sensor 1, pressing force read by sensor 2, pressing force read by sensor 3, pressing force read by sensor 4 are stored in memory.

The loop 302-303 is terminated by receiving in step 304 a stopping command to stop measurement of exercise set. The stopping command may be initiated by the user using an interface of an application executed at the data processing device, e.g., by pressing a dedicated EXERCISE STOP button. Alternatively, the stopping command may be initiated by a haptic command input to the measurement module 130, e.g., a tap or double tap command. The data processing device may play an audible sound informing the user about receipt of the stopping command. Alternatively, the loop 302-303 may be terminated upon expiry of a predetermined time limit that is longer than the expected time to perform the exercise, in order to stop measurements and save battery life in cases where the user has indicated starting of an exercise but did not issue a stopping command. Alternatively, the loop 302-303 may be terminated upon detecting measurement values that indicate that the exercise has ended, e.g., by deviation from a pattern that corresponds to a typical pattern for a particular exercise.

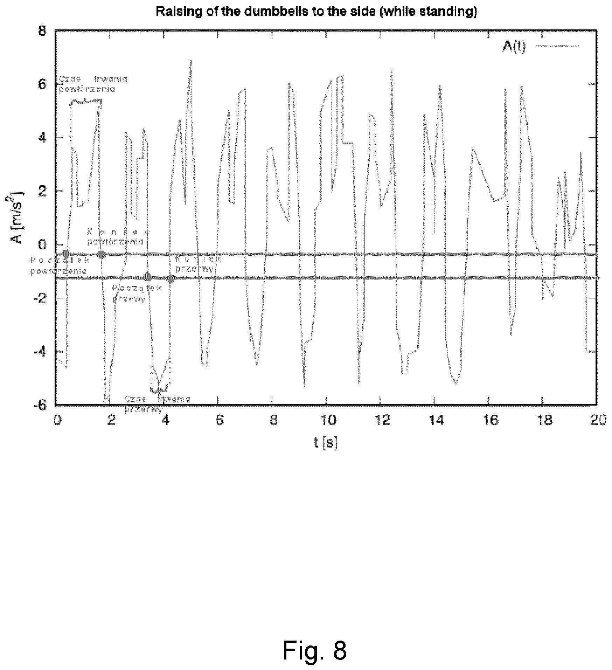

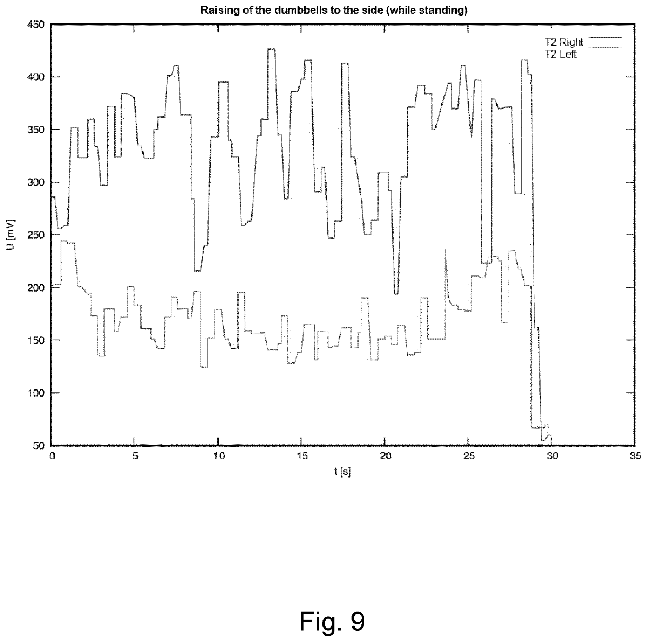

In step 305 the data read from the sensors is pre-processed, as explained in details with reference to Figs. 8 and 9.

In step 306 the pre-processed data is sent via the wireless interface 131B from the measurement module 130 do the data processing device. By sending pre-processed data instead of all measurement data collected by the sensors, battery life of the measurement module 130 is extended as the pre-processing takes less power than transmission of large amount of data.

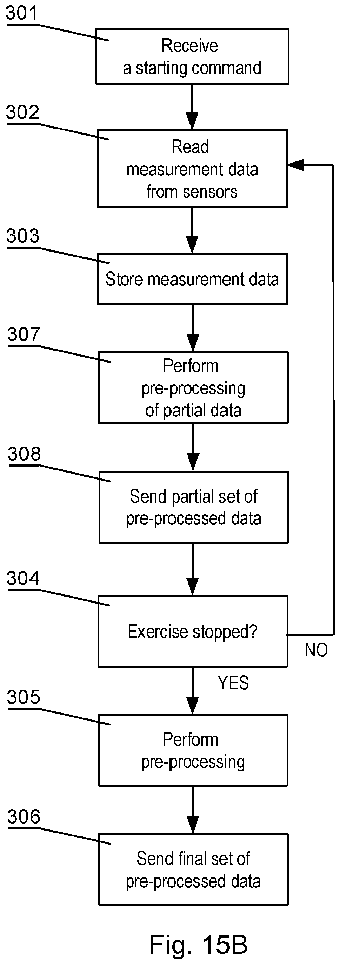

Fig. 15B shows a second embodiment of the procedure for reading and pre-processing measurement data, which differs from the procedure of Fig. 15A in that it comprises additional steps 307, 308 of performing pre-processing of partial data and sending partial pre-processed data while the exercise is performed (e.g. after each collection of new measurement data (i.e. with the same frequency as reading measurement data) or with a lower frequency than reading measurement data (e.g. every 10 measurements)). The pre-processing is performed on the data already available, in a way described for steps 305, 306 of Fig. 15A. Next, after the exercise is stopped, pre-processing of final (all available) data is performed in step 305 and final data are sent in step 306.

For example, if the wireless interfaces 131B, 141 operate in accordance with the BLE (Bluetooth Low Energy) standard, the communication may be performed using GAP and GATT protocol (GAP used to determine method of communication: active/passive/data and GATT used to determine service and characteristics attributes). For example, a start and stop command may be transmitted using a WRITE method and pre-processed data can be read using READ method. A NOTIFY method may be used to send pre-processed data periodically or after the end of the exercise.

For example, the following services may be defined:

TENSOR SERVICE - service containing raw data from each measurement of each pressing force sensor

Internal characteristics:

    TENSOR_CHANNEL_1 - raw data from first sensor
    TENSOR_CHANNEL_2 - raw data from second sensor
    TENSOR_CHANNEL_3 - raw data from third sensor
    TENSOR_CHANNEL_4 - raw data from fourth sensor

MOVEMENT SERVICE - service including raw accelerometer and gyroscope data

Internal characteristics:

    ACELEROMETER_X - raw accelerometer measurements in X axis
    ACELEROMETER_Y - raw accelerometer measurements in Y axis
    ACELEROMETER_Z - raw accelerometer measurements in Z axis
    GYROSCOPE_X - raw gyroscope measurements in X axis
    GYROSCOPE_Y - raw gyroscope measurements in Y axis
    GYROSCOPE Z - raw gyroscope measurements in Z axis
    DOUBLE_TAP - detection of a double tap haptic gesture

AGGREGATED DATA SERVICE - service including pre-processed data

Internal characteristics:

    AGGREGATED_TENSOR_DATA - pre-processed data from all pressing force sensors
    AGGREGATED ACCELERO_DATA - pre-processed data from accelerometer
    AGGREGATED_ALGO_COMMANDS_DATA - characteristic for sending starting and stopping commands
    AGGREGATED ALGORITHMS DATA - data including information on repetitions_number, start_datetime, end_datetime
    AGGREGATED BALANCE _DATA - data of total force detected by the sensor, to calculate balance of that hand.

Fig. 8 shows an example of measurement data from accelerometers during an exercise of lifting a dumbbell sideways while standing.

Based on such measurement data, the pre-processing in step 305 can be performed as explained below, to determine the number of repetitions, the duration of each repetition and the length of the intervals between repetitions.

After starting the set, by using the data from the accelerometer for each measurement, the device calculates the resultant length of the acceleration vector according to the following formula:

$$A_w = \sqrt{A_x^2 + A_y^2 + A_z^2}$$

The $A_w$ values are stored in the local memory of the controller 131A. During pre-processing, the string of $A_w$ values is averaged using the following moving average formula:

$$A_{sr} = \frac{A_{w0} + A_{w1} + \cdots + A_{wn}}{n};$$

where n is the number of periods determined empirically in order to minimize the measurement errors of the accelerometer.

The algorithm monitors the $A_{sr}$ values and if its absolute value exceeds the established $A_{pr}$ threshold (determined empirically), it will mark the given measuring point as the start of the repetition. All subsequent $A_{sr}$ values greater than the $A_{pr}$ threshold are taken as values during the repetition duration. Likewise, the point at which $A_{sr}$ falls below the $A_{pr}$ threshold is considered the end of the repetition.

In general, the data readings from the left glove and right glove accelerometers are substantially similar, so that only a single graph is shown in Fig. 8. In addition, the graph (read values from accelerometers) allows to determine the dynamics of the exercises/repetitions. For example, the falling dynamics of repetitions may be a sign of increasing user tiredness.

In addition, by comparing the readings of a given set with the previous sets or the typical values for a given exercise, it can be determined whether the user is improving or deteriorating in the exercises. As a result, for example, it can generate a message for the user: "You performed much worse in this set, you are already tired."

Fig. 9 shows an exemplary reading of data from a single pressing force sensor on the left and right gloves during an exercise involving lifting a dumbbell sideways while standing.

By comparing the data from the pressing force sensor 132 (strain gauge) data, it is possible to calculate the "total force" applied to the weight by summing (integrating) the area under the plots from each pressing force sensor 132 and comparing the left and right glove. In the above graph, the area under the graph from the right glove is larger than the area under the graph from the left glove, which means that the share of the right hand and at the same time the balance for the right hand is greater.

Therefore, the data from the pressing force sensors 132 is pre-processed in step 305 as follows. First, the stored measurement values (e.g., voltage readings from the pressing force sensors converted to a value of pressing force) are converted to a standardized scale using a well-known formula similar to Z-score normalization, such that:

$$Tj_{si} = \frac{Tj_i - \mu}{\sigma}$$

wherein:

$T_{jsi}$ is the standardized value,

j is a reference for the sensor 132 (e.g., j is 1, 2, 3, 4 - if 4 sensors are present),

$T_{ji}$ is a measurement value before standardization,

i is an index of measurement (e.g., for 10 Hz and 100 seconds there are 1000 measurements),

μ is an average value of all i measurements in the set,

σ is a standard deviation within all i measurements in the set.

Next, an integral is calculated based on equation:

$$\int_0^n Tj_s \, dn.$$

wherein n is the number of measurements, to calculate the standardized total force measured by all sensors 1-4 of the particular glove.

The total force measured is transmitted as a pre-processed data from that glove in step 306.

In addition to the measurement of the total force by all sensors, a total force can be calculated for each sensor individually and transmitted as the pre-processed data from that glove in step 306.

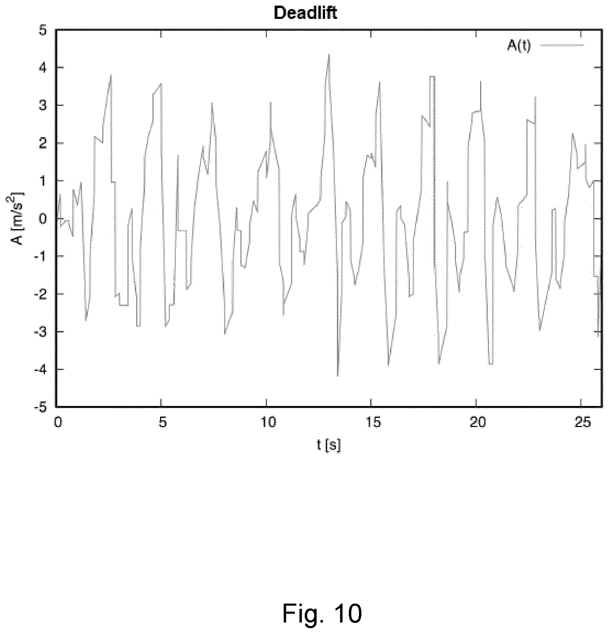

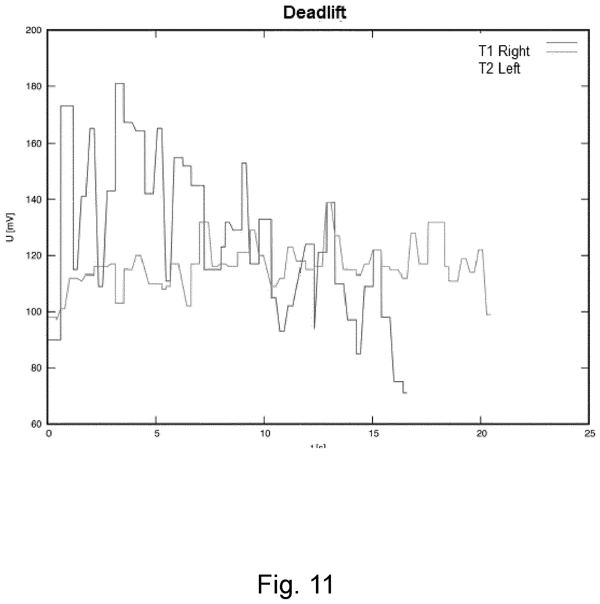

Analogously to Figs. 8 and 9, Fig. 10 respectively shows an exemplary reading of data from the accelerometers during a deadlift exercise. Fig. 11 shows an exemplary reading of data from a single pressing force sensor on the left and right gloves during a deadlift exercise. From the read data, the system algorithm counts the number of repetitive cycles as the number of repetitions along with the duration of each repetition. The results of the analysis of the change in duration of each repetition over several sets are presented to the user. The pressing force values from the pressing force sensors for the left and right hands are quite even in this case. It can be seen a very even balance between the left and right hands, which is below 5% (in particular, in the middle of the graph).

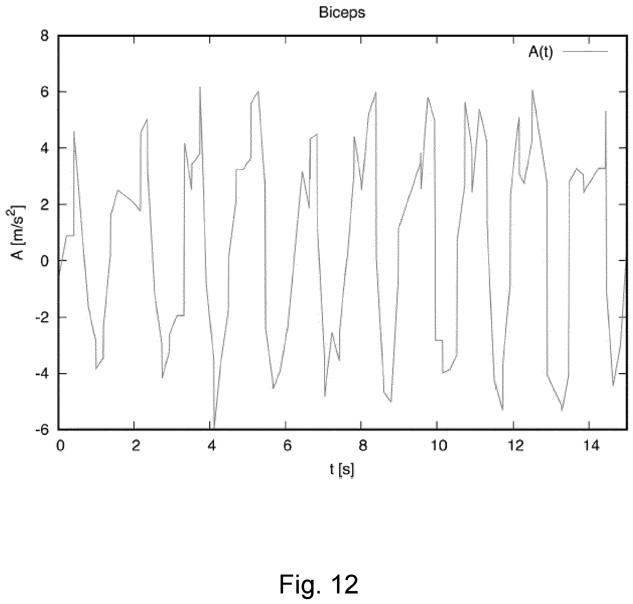

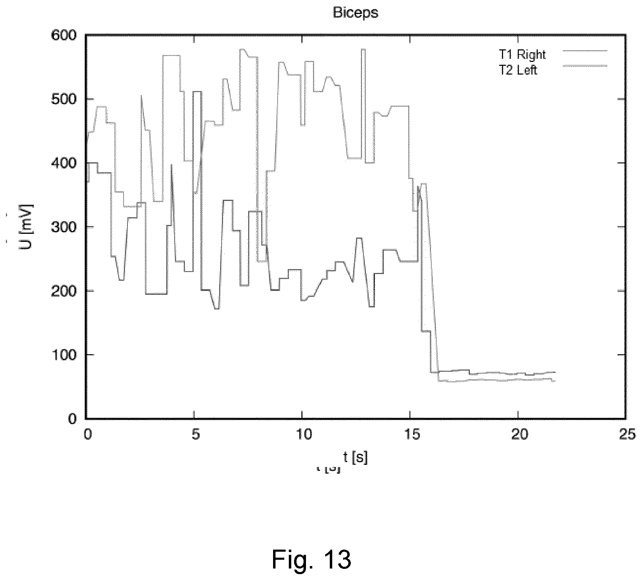

Analogous to Figs. 8-11, Fig. 12 shows an exemplary reading of the accelerometer data during a dumbbell lifting exercise for a ten repetitions biceps exercise. Fig. 13 shows an exemplary reading of data from a single pressing force sensor on the left and right gloves during a dumbbell lifting exercise for a biceps exercise, from which a strong dominance of the left hand can be observed.

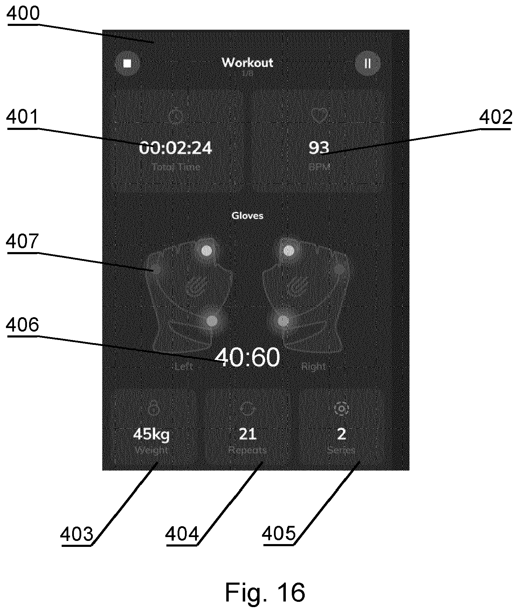

After each exercise set (if performing procedure of Fig. 15A), or during performing the exercise (if performing procedure of Fig. 15B), the system presents a summary message, for example as shown in Fig. 16. The summary message 400 may include information on the duration of the set 401, the hear rate 402 after completion of the set, the weight lifted 403, number of performed repetitions 404, number of series 405, a balance percentage of left to right hand 406 (e.g. 40:60) calculated on the basis of total force read by the left and right glove. If the pre-processed data from each glove includes total force data per each sensor, the summary message 400 may further comprise a visual representation 407 of the gloves with position of the sensors, wherein each sensor is represented by a color corresponding to the amount of force measured, thereby the user may easily determine which sensors had most impact on the imbalance between the hands. In addition, after finishing the training, full statistics on the course of training are displayed (number of exercises, repetitions, sets), the level of fatigue, information about the dominant hand, the quantitative assessment of the training. For example, the quality of the entire training can be expressed as a percentage, compared to previously collected statistics.

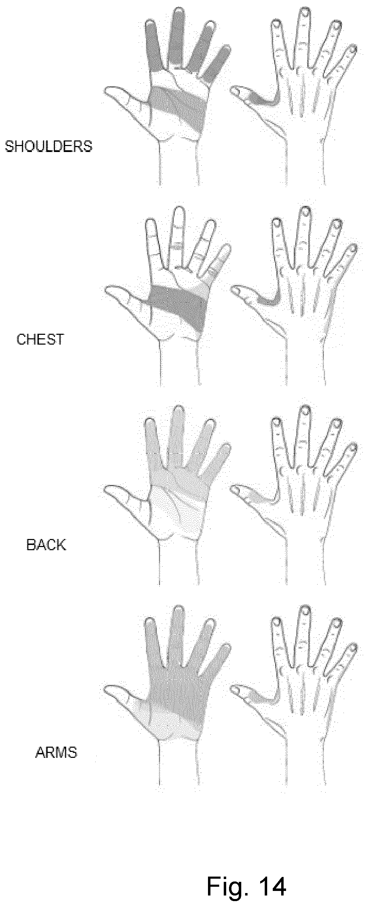

Figure 14 shows the distribution of forces acting on the hand when performing certain types of exercise to indicate which sensors play the most important role in monitoring the exercise. As a result, it is possible to present a hand heat map that is related to the pressing force distribution.

In particular, during exercise, the system presents information on the number of repetitions, the current heart rate and pulse zone, blood saturation, as well as it can graphically present four points on the hand, corresponding to four pressing force sensors on the gloves, which visually show the pressing force value and a light signal (green, yellow, red) symbolizing the current deviations and irregularities of the handle.

While the invention has been described with respect to an embodiment, it will be appreciated that many variations, modifications, and other applications of that embodiment may be made. Therefore, the claimed invention as recited in the claims that follow is not limited to the embodiment described herein.

**Claims**

1. A method for monitoring of exercises performed by a user, the method comprising:

   - providing the user with a pair of gloves (110, 120) including a left glove (110) and a right glove (120), wherein each of the gloves (110, 120) comprises a measurement module (130), the measurement module (130) comprising:

     - a three-axis accelerometer (133);
     - a three-axis gyroscope (134); at least four pressing force sensors (132) arranged at predetermined positions, wherein the positions of the pressing force sensors on the left glove (110) are symmetrical with respect to the positions of the pressing force sensors on the right glove (120); and
     - a controller (131A) for collecting measurement data from the sensors (132) and the accelerometer (133);
     - a glove wireless data transmission module (131B);

   - providing a data processing device (140), the data processing device (140) comprising:

     - a wireless data transmission module (141) communicatively coupled to the glove wireless data transmission modules (131B) of each of the gloves (110, 120);
     - a data processing system (142); and
     - a signaling system (143);

   - performing the following steps, in real time, while the user performs the exercise while wearing the gloves (110, 120):

     - at each glove, during performance of the exercise set, reading and pre-processing (201) measurement data from the sensors (132) and accelerometer (133) in the gloves (110, 120) to determine at least a standardized total force measured by the sensors (132) during performance of the exercise set;
     - by means of the glove wireless data transmission modules (131B) of each of the gloves, transmitting (202) the pre-processed data to the wireless data transmission module (141) of the data processing device (140);
     - by means of the data processing system (142), processing (203) the transmitted data to analyze differences between data from the sensors of the left glove (110) and the data from the sensors of the right glove (120); and
     - by means of the signaling system (143), providing a feedback (204) to the user, wherein the feedback indicates at least a balance between the standardized total force measured at the left glove (110) and the right glove (120).

2. The method according to claim 1, wherein the data processing device (140) is a smartphone operating an application executing the functions of the data processing circuit (142).

3. The method according to claim 1, wherein the data processing device (140) is a tablet or a TV set operating an application performing the functions of the data processing circuit (142).

4. The method according to claim 1 or 2, wherein the signaling system (143) comprises a display.

5. The method according to any of the preceding claims, wherein the signaling system (143) comprises an audio emitter.

6. The method according to any of the preceding claims wherein the signaling system (143) comprises a vibrating element embedded within the glove (110, 120).

7. The method according to any of the preceding claims wherein the glove (110, 120) comprises a pulse oximeter configured to measure the pulse and blood oxygen saturation of the user.

8. The method according to any of the preceding claims wherein each glove (110, 120) comprises four pressing force sensors (132) in the following arrangement: a first sensor arranged at the middle finger near the middle phalanx bone, a second sensor arranged at the index finger near the proximal phalanx bone, a third sensor arranged at the second metacarpal bone and a fourth sensor arranged at the fifth metacarpal bone, when the glove is positioned on the hand of the user.

9. The method according to any one of the preceding claims, further comprising: before reading (201) and pre-processing measurement data from the sensors (132) and the accelerometer (133), detecting a finger tap (301) on the glove; and in response to detecting the finger tap, starting reading of the transmitted data from the pressing sensors (132) and the accelerometer (133) and continuing the reading until detecting another finger tap (304).

10. The method according to any of the preceding claims wherein at least one glove (110, 120) is provided with a pedometer.

11. The method according to any one of the preceding claims wherein the measurement module (130) comprises a computing module for preprocessing the sensor data.

12. The method according to any of previous claims, wherein reading (201) and pre-processing measurement data from the sensors (132) comprises storing (303) measurement data read from the sensors (132) and pre-processing (305) stored measurement data after the exercise is stopped (304) to determine a total pressing force measured by the sensors (132) and sending (306) a final set of pre-processed data.

13. The method according to any of previous claims, wherein reading (201) and pre-processing measurement data from the sensors (132) comprises storing (303) measurement data read from the sensors (132) and repetitively pre-processing (307) stored measurement data to determine a total pressing force measured by the sensors (132) and sending (308) a partial set of pre-processed data.

14. A system for monitoring of exercises performed in gloves, comprising a pair of gloves and a data processing device (140) having a structure and configured to operate according to any of claims 1-11.

Fig. 1

201 — Read and pre-process data from sensors

202 — Send pre-processed data to the data processing device

203 — Process read data

204 — Analyze data and present differences in signals

Fig. 2

110,
120

130

Fig. 3A

132

Fig. 3B

138

136

137

152

Fig. 4

139

138

137

136

135

Fig. 5

Fig. 6A

Fig. 6B

136

1362

1363

1361

Fig. 6C

Fig. 7A

Fig. 7B

Fig. 8

Fig. 9

Fig. 10

Fig. 11

Fig. 12

Fig. 13

SHOULDERS

CHEST

BACK

ARMS

Fig. 14

301 — Receive
a starting command

302 — Read
measurement data
from sensors

303 — Store measurement data

304 — Exercise stopped?

NO

YES

305 — Perform pre-processing

306 — Send pre-processed data

Fig. 15A

301 — Receive
a starting command

302 — Read
measurement data
from sensors

303 — Store measurement data

307 — Perform
pre-processing
of partial data

308 — Send partial set of
pre-processed data

304 — Exercise stopped?

NO

YES

305 — Perform
pre-processing

306 — Send final set of
pre-processed data

Fig. 15B

400

401                                                      402

Workout

00:02:24                        93
Total Time                       BPM

Gloves

407

406
40:60
Left                    Right

45kg            21              2
Weight         Repeats        Series

403            404            405

Fig. 16

Europäisches
Patentamt

European
Patent Office

Office européen
des brevets

# EUROPEAN SEARCH REPORT

Application Number

EP 22 18 4190

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | US 2018/193699 A1 (DEBATES SCOTT P [US] ET AL) 12 July 2018 (2018-07-12) | 1-8, 10-14 | INV. G16H20/30 |
| A | * [0008], [0016]-[0017], [0019]-[0023], [0026], [0028], [0030]-[0031], [0037]-[0038], [0040]-[0042], [0046], [0052]; figures 1-3 * | 9 | A63B24/00 A61B5/00 A61B5/11 G06K9/00 G06V40/20 |
| Y | US 2019/099123 A1 (ZAMBRISKI SHARON ANN [US] ET AL) 4 April 2019 (2019-04-04) | 1-8, 10-14 | |
| A | * [0005], [0018]-[0021], [0025]-[0026], [0036], [0039]; figures 1-3, 6-7 * * [0006], [0028]; figure 9 * | 9 | |
| Y | WO 2018/129175 A1 (ACTIVBODY INC [US]) 12 July 2018 (2018-07-12) * [0003]-[0007]; figures 1A, 1B, 2A * | 1-8, 10-14 | |
| Y | US 2018/333079 A1 (SZÉKELY LAJOS [HU] ET AL) 22 November 2018 (2018-11-22) * [0011], [0037], [0045], [0047]-[0049]; figures 1, 6, 8-9 * | 8 | TECHNICAL FIELDS SEARCHED (IPC) G16H A63B G06K A61B G06V |
| A | CN 111 752 393 A (LI FEIXIANG) 9 October 2020 (2020-10-09) * "Summary of the invention"; figure 5 * | 9 | |

The present search report has been drawn up for all claims

| Place of search | Date of completion of the search | Examiner |
|---|---|---|
| Munich | 9 November 2022 | Werner, Andreas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ...................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 18 4190

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2022

| Patent document cited in search report | Publication date | Patent family member(s) | | Publication date |
|---|---|---|---|---|
| US 2018193699 A1 | 12-07-2018 | NONE | | |
| US 2019099123 A1 | 04-04-2019 | NONE | | |
| WO 2018129175 A1 | 12-07-2018 | CN | 110337270 A | 15-10-2019 |
| | | EP | 3565468 A1 | 13-11-2019 |
| | | JP | 2020509343 A | 26-03-2020 |
| | | JP | 2022126676 A | 30-08-2022 |
| | | US | 2018188122 A1 | 05-07-2018 |
| | | US | 2019265115 A1 | 29-08-2019 |
| | | WO | 2018129175 A1 | 12-07-2018 |
| US 2018333079 A1 | 22-11-2018 | BR | 112018009481 A2 | 05-02-2019 |
| | | CA | 3005000 A1 | 18-05-2017 |
| | | EP | 3401873 A1 | 14-11-2018 |
| | | IL | 259280 A | 31-07-2018 |
| | | JP | 6837484 B2 | 03-03-2021 |
| | | JP | 2019500083 A | 10-01-2019 |
| | | US | 2018333079 A1 | 22-11-2018 |
| | | WO | 2017081497 A1 | 18-05-2017 |
| | | ZA | 201803835 B | 25-09-2019 |
| CN 111752393 A | 09-10-2020 | NONE | | |

EPO FORM P0459

For more details about this annex : see Official Journal of the European Patent Office, No. 12/82

**EP 4 116 990 A1**

**Patent documents cited in the description**

- US 2017216665 A1
- US 20150196803 A1
- US 2018193699 A1